# NOUVEAU FASCICULE DE BREVET EUROPEEN

(11) **EP 1 066 016 B2**
(45) Date de publication et mention de la décision concernant l'opposition: **24.09.2008**
(45) Mention de la délivrance du brevet: 06.07.2005
(21) Numéro de dépôt: 99902604.0
(22) Date de dépôt: 05.02.1999
(51) Int. Cl.: A61K 8/44, A61Q 5/12, A61K 8/88, A61Q 5/00, A61Q 5/02

(54) **DERIVES DE POLYAMINO-ACIDES ET LEUR UTILISATION DANS DES COMPOSITIONS DE TRAITEMENT DES FIBRES KERATINIQUES**
POLYAMINOSÄUREDERIVATE UND IHRE VERWENDUNG IN ZUSAMMENSETZUNGEN ZUR BEHANDLUNG VON KERATINISCHEN FASERN
POLY-AMINOACID DERIVATIVES AND THEIR USE IN COMPOSITIONS FOR TREATING KERATINOUS FIBRES

(30) Priorité: 31.03.1998 FR 9803965
(43) Date de publication de la demande: 10.01.2001
(73) Titulaire: L'ORÉAL, 75008 Paris (FR)
(72) Inventeur: PHILIPPE, Michel, F-91320 Wissous (FR); BLAISE, Christian, F-94160 Saint Mandé (FR)
(74) Mandataire: Martin-Charbonneau, Virginie
(86) Numéro de dépôt international: PCT/FR1999/000256
(87) Numéro de publication internationale: WO 1999/049837

(56) Documents cités:
- DE-A- 2 653 560
- FR-A- 2 533 209
- FR-A- 2 533 222
- DATABASE WPI Week 8916 Derwent Publications Ltd., London, GB; AN 89-117235 XP002092501 "Cosmetic material compsn. - comprises compounded fibroin peptide ester" & JP 01 061412 A (SEIWA KASEI) , 8 mars 1989
- T. SHIMIZU ET AL: "Self-assembling properties of synthetic peptidic lipids" BIOCHIMICA ET BIOPHYSICA ACTA, vol. 1147, 1993, pages 50-58, XP002092500
- DATABASE WPI Week 9235 Derwent Publications Ltd., London, GB; AN 92-288870 XP002092502 "Moisture retaining agent for cosmetic material - comprises poly-aminoacid comprising acidic and neutral aminoacid(s) and alkali metal and mono-, di- or triethanolamine salts of carboxylic acids in side chains" & JP 04 198114 A (AJINOMOTO) , 17 juillet 1992 cité dans la demande
- DATABASE WPI Week 9749 Derwent Publications Ltd., London, GB; AN 97-532840 XP002092503 "Composite particles used for cosmetics and medicines, etc." & JP 09 255778 A (NIPON GOSEI GOMU) , 30 septembre 1997
- DATABASE WPI Week 9516 Derwent Publications Ltd., London, GB; AN 95-118690 XP002092504 "New N-carboxy-aminoacid anhydride - used in mfr. of high mol. wt. polypeptide(s)" & JP 07 041467 A (AJINOMOTO) , 2 octobre 1995 cité dans la demande
- DATABASE WPI Week 9016 Derwent Publications Ltd., London, GB; AN 90-119695 XP002092505 "Cationic ampipathic substances contg. oligopeptide sites - have affinity for both aq. solvent and organic solvent, used as emulsifiers, stabilizer, dispersant, etc." & JP 02 069498 A (AGENCY OF IND. SCI & TECHNOLOGY), 8 mars 1990
- DATABASE WPI Week 9847 Derwent Publications Ltd., London, GB; AN 98-551188 XP002092506 "Composition comprising oligopeptide alkylamide derivative - has excellent stability" & JP 10 245396 A (POLA CHEM) , 14 septembre 1998
- DATABASE WPI Week 9902 Derwent Publications Ltd., London, GB; AN 99-018385 XP002092507 "Cystein containing peptide(s) for treating hair - prepared by introducing cysteine in hydrolysed naturally derived protein by amide binding of amino group in hydrolysate and carboxy group of cysteine" & JP 10 287697 A (SEIWA KASEI) , 27 octobre 1998
- (Die Nutrilan-Reihe, Verkaufsbrochüre der Henkel KGaA, gedruckt gemäss letzter Seite, letzte Zeile 1086)

## Description

La présente invention a pour objet l'utilisation de dérivés de polyamino-acides dans une composition cosmétique pour le renforcement et le soin des fibres kératiniques en particulier des cheveux.

La présente invention a également pour objet un procédé de traitement "direct" ou "in situ" pour le renforcement et le soin des fibres kératiniques.
Par procédé "direct", on entend le traitement des fibres par application sur les fibres d'une composition cosmétique contenant, dans un véhicule cosmétiquement approprié, au moins un dérivé de polyaminc-acide préalablement synthétisé.
Par procédé "in situ", on entend le traitement des fibres à l'aide de précurseurs donnant naissance, au sein de celles-ci, à un dérivé de polyamine-acide.

Différentes classes de polyamino-acides ont été décrites, et leur utilisation est bien connue et largement pratiquée notamment pour leurs propriétés d'hydratation.
A ce sujet, il a été décrit dans la demande japonaise JP-07/041,467 une classe de polyamino-acides de poids moléculaire élevé constitué essentiellement de cystéine, ainsi que le procédé de préparation de ces polyamino-acides.
Une classe de polyamino-acides caractérisée par la présence de fonctions thiols et/ou disulfures a également été décrite dans la demande japonaise JP-06/248,072. Ces polyamino-acides réagissent avec les liaisons thiols de la kérattne formant ainsi des ponts disulfures, ce qui permet de rehausser les qualités de brillance et de coloration de la chevelure.
Une autre classe de polyamino-acides a été décrite dans la demande japonaise JP-04/198,114, ceux-ci étant constitués essentiellement d'acides aminés à chaînes neutres et acides et sont utilisés de manière générale en tant qu'agent hydratant.
Enfin, dans la demande française FR-2533209 sont décrits des lipopeptides amphipatiques constitués d'une chaîne peptidique hydrophile et d'une chaîne hydrophobe de 8 à 24 atomes de carbone, ainsi que leur utilisation comme agents émulsifiants de milieux non miscibles ou pour l'obtention de cristaux liquides.

Après de nombreuses études effectuées sur diverses classes de polyamino-acides, on a constaté de façon surprenante et inattendue qu'une classe particulière de dérivés de polyamino-acides présentait d'importantes propriétés de renforcement des fibres kératiniques, résultant de la formation d'un dépôt substantiel de matériau polypeptidique à la surface des fibres kératiniques.

L'utilisation de ces dérivés de polyamino-acides dans des compositions cosmétiques capillaires permet par ailleurs d'améliorer la tenue et le volume des cheveux.

La présente invention a donc pour objet l'utilisation, dans une composition cosmétique pour le renforcement des fibres kératiniques, d'au moins un dérivé de polyamino-acide de formule générale (I) suivante : 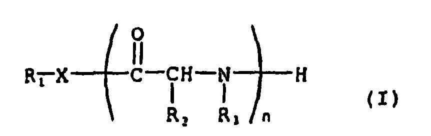 dans laquelle:
X est -O-, -S- ou -NR₃
R₁ représente :
   (ii) un radical alkyle, linéaire ou ramifié, saturé ou insaturé, en C₁-C₄₀, éventuellement substitué par au moins un hydroxy ou un radical 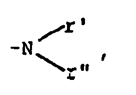 et/ou éventuellement interrompu par au moins un hétéroatome choisi parmi N, O ou Si, r' et r", identiques ou différents, étant un atome d'hydrogène ou un radical alkyle en C₁-C₆,
   (iii) 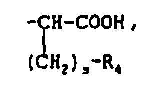 s étant 0 à 4
   (iv) 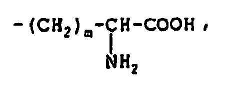 m étant 3 à 5,
R₂ représente un atome d'hydrogène, un radical alkyle en C₁-C₈, -CH₂C₆H₅, -CH₂C₆H₅p-OH, -CH₂OH, -CHOH-CH₃ ou -(CH₂)ᵣ-NH₂, ₜ étant 3 à 5,
R₃ représente un atome d'hydrogène ou un radical alkyle en C₁-C₆,
R₄ représente un atome d'hydrogène, -NH₂, -OH, -SH, -CHOHCH₃, -CONH₂, 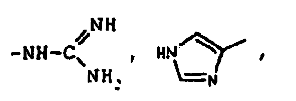 -C₆H₅ ou -C₆H₅p-OH, et
n est un nombre moyen supérieur à 1 tel que le poids moléculaire du dérivé de polyamino-acide soit compris entre 200 et 200.000, l'unité répétitive étant soit identique pour un même dérivé, soit différente, R₂ et/ou R₃ prenant alors au moins l'une des autres significations données pour ces radicaux, ou d'un sel dudit dérivé de polyamino-acide.

Les polyamino-acides de formule (I) sont pour certains connus et d'autres nouveaux.

Leur procédé d'obtention consiste en une réaction de polycondensation entre au moins un N-carboxyanhydride de formule : 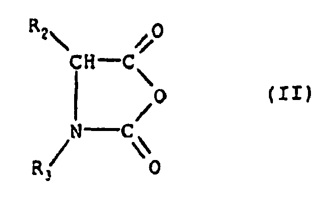 dans laquelle :
R₂ et R₃ ont les mêmes significations que celles données ci-dessus pour la formule (I) et un composé nucléophile de formule (III) :

   R₁-XH (III)

   dans laquelle R₁ et X ont les mêmes significations que celles données ci-dessus pour la formule (I).

Les N-carboxyanhydrides de formule (II) sont préparés par les méthodes usuelles en faisant réagir un D et/ou L α-amino-acide avec du phosgène dans un solvant inerte tel que le dioxane ou le tétrahydrofurane (THF).

La réaction de polycondensation est généralement réalisée à une température comprise entre environ 0 et 120°C dans un solvant inerte choisi parmi le benzène, le toluène, le chlorobenzène, le dichloroéthane, le diméthylacétamide (DMAC), le diméthylformamide (DMF), la méthyl éthyl cetone (MEC), les éthers aliphatiques tels que l'éther éthylique, l'éther isopropylique, le tert-butyl méthyl éther, et les éthers cycliques tels que le tétrahydrofurane et le dioxane. Toutefois, le solvant de réaction peut être éventuellement de l'eau.

Dès le début de la réaction, on peut constater la formation d'un dégagement plus ou moins important de CO₂.

Après refroidissement éventuel du milieu réactionnel, on évapore le solvant puis on sèche sous vide le dérivé de polyamino-acide obtenu.

Suivant la pureté obtenue, une étape de purification telle qu'une précipitation ou une cristallisation du brut obtenu peut être effectuée.

Dans le procédé de préparation du dérivé de polyamino-acide tel que décrit ci-dessus, le composé nucléophile joue le rôle d'initiateur de la réaction de polycondensation.

Selon un mode de réalisation préfére, la quantité en mole du composé nucléophile de formule (III) est de 1/2-1/3000, de préférence 1/5-1/2000 par mole du N-carboxyanhydride de formule (II).

Parmi les N-carboxyanhydrides de formule (II), on peut notamment citer :
- la N-carboxyanhydride glycine,
- la N-carboxyanhydride sarcosine,
- la N-carboxyanhydride thréonine,
- la N-carboxyanhydride sérine,
- la N-carboxyanhydride valine,
- la N-carboxyanhydride norvaline,
- la N-carboxyanhydride isoleucine,
- la N-carboxyanhydride leucine,
- la N-carboxyanhydride norleucine,
- la N-carboxyanhydride lysine,
- la N-carboxyanhydride phénylalanine,
- la N-carboxyanhydride tyrosine, et
les N-carboxyanhydrides ci-dessus éventuellement sous forme protégée.

Parmi les composés nucléophiles de formule (III), on peut notamment citer les amines telles que le 2-amino octadécane-1,3 diol, la 2-octyl dodécylamine, l'hexylamine, l'oléylamine, la glucamine, la lysine, l'arginine, l'histidine, et la glutamine, les alcools sous forme d'alcoolate tel que le méthanolate de sodium, et les thiols tels que la cystéine.

Les compositions selon l'invention peuvent se présenter sous diverses formes et contiennent le dérivé polyamino-acide en une proportion généralement comprise entre 0,001 et 30 %, et de préférence entre 0,01 et 15 % en poids par rapport au poids total de la composition.

Selon un premier mode de réalisation des compositions selon l'invention, celles-ci se présentent sous forme d'un shampooing ou d'une composition de soins avant ou après shampooing pour le renforcement des fibres kératiniques.

Le véhicule cosmétiquement approprié de ces compositions est soit un milieu aqueux soit un milieu hydroalcoolique contenant un alcool tel que l'éthanol ou l'isopropanol.

Les compositions sous forme d'un shampooing contiennent en outre au moins un agent tensioactif anionique, non-ionique, zwittérionique, amphotère ou cationique.

La proportion en agent tensioactif est généralement comprise entre 0,01 et 50 % en poids, mais de préférence entre 0,05 et 30 % en poids par rapport au poids total de la composition.

Lorsque l'on utilise un agent tensioactif du type non-ionique, celui-ci est généralement utilisé en une proportion comprise entre 0,1 et 40 % en poids, et de préférence entre 1 et 20 % en poids par rapport au poids total de la composition.

Les agents tensioactifs du type cationique, en raison de leur plus faible pouvoir détergent, sont plus particulièrement utilisés dans les compositions de soins avant ou après shampooing.

Les tensioactifs susceptibles d'être utilisés dans les compositions selon ce mode de réalisation sont bien connus de l'état de la technique et ont notamment été décrits dans le brevet FR-2,728,163.

Ces compositions de soins avant ou après shampooing peuvent en outre contenir un agent conditionneur.

Comme agents conditionneurs pouvant être utilisés dans les compositions selon l'invention, on peut citer notamment les huiles naturelles hydrogénées ou non, les huiles synthétiques hydrocarbonées, cycliques ou aliphatiques, linéaires ou ramifiées, saturées ou insaturées, telles que par exemples les poly α-oléfines, en particulier les polydécènes et polyisobutènes, les huiles de silicone volatiles ou non, organo-modifiées ou non, solubles ou non, les huiles fluorées ou perfluorées, les esters gras, les esters d'alcools polyhydriques et les glycérides.

On peut également utiliser, comme agents conditionneurs, des cires synthétiques ou naturelles, des gommes et résines de silicone, des protéines ou des hydrolysats de protéines quaternisés ou non ou un mélange de ces divers agents.

Les compositions selon ce premier mode de réalisation peuvent également contenir au moins un additif cosmétiquement ou dermatologiquement acceptable choisi parmi un agent épaississant, un polymère de type cationique, anionique, non-ionique ou amphotère, un filtre solaire, un céramide, un α-hydroxyacide, un agent conservateur, un agent anti-microbien, un agent anti-pelliculaire, un agent nacrant, un agent colorant, un parfum, un électrolyte ou un agent de mise en suspension.

Les additifs sont généralement présents dans les compositions selon l'invention en une proportion comprise entre 0,01 et 20 % en poids, et de préférence entre 0,02 et 10 % en poids par rapport au poids total de la composition.

Le pH des compositions selon l'invention est généralement inférieur à 7 et de préférence compris entre 3 et 4,5.

Selon un second mode de réalisation des compositions selon l'invention, celles-ci se présentent sous forme d'une composition pour la formation d'une mousse de type anhydre ou aqueux.

Lorsque la composition de mousse est de type anhydre, celle-ci comprend une phase grasse en tant que véhicule cosmétiquement acceptable, un agent moussant et un agent propulseur.

La phase grasse peut être constituée d'une huile cosmétique ou de l'association d'une huile et d'au moins une cire.

Parmi les huiles et les cires susceptibles d'être utilisées, on peut notamment citer celles d'origine végétale, animale, minérale ou synthétique. Ces huiles et cires sont bien connus de l'état de la technique et sont notamment décrits dans le brevet FR-2,668,927.

L'huile cosmétique ou le mélange d'huile et de cire est généralement présent en une proportion comprise entre 20 et 99 % en poids, mais de préférence entre 25 et 85 % en poids par rapport au poids total de la phase grasse.

Lorsque la composition de mousse est de type aqueux, celle-ci comprend une solution aqueuse ou un mélange hydroalcoolique en tant que véhicule cosmétiquement approprié, un agent moussant et un agent propulseur.

Le mélange hydroalcoolique peut contenir par exemple un alcool en C₁-C₄ tel que l'éthanol ou l'isopropanol.

Lorsque l'on utilise une solution hydroalcoolique, la proportion en alcool n'excède pas environ 50 %, et est de préférence inférieure à 30 % en poids par rapport au poids total de la composition.

Parmi les agents moussants ou tensioactifs susceptibles d'être utilisés, on peut citer entre autres les tensioactifs anioniques, non-ioniques, zwittérioniques, amphotères ou cationiques. On peut également citer les polymères cationiques ou anioniques tels que ceux décrits dans la demande de brevet FR-8207996, ou encore l'association de polymères cationiques et amphotères telle que décrite dans la demande de brevet FR-9602125.

La proportion en agent moussant peut être comprise entre 0,05 et 20 % en poids, de préférence entre 0,2 et 10 % en poids, et plus particulièrement comprise entre 0,25 et 5 % en poids par rapport au poids total de la composition.

Les compositions de mousse de type aqueux peuvent également contenir un agent plastifiant en une proportion comprise entre 0,01 et 16 % en poids par rapport au poids total de la composition.

Les agents plastifiants susceptibles d'être utilisés sont bien connus et sont notamment décrits dans le brevet FR-2,719,995.

Parmi les ingrédients susceptibles d'être ajoutés dans les compositions de mousse, on peut citer les colorants ayant pour fonction de colorer la composition elle-même ou les cheveux, les agents conservateurs, les agents séquestrants, les agents régulateurs du pH, les parfums, les vitamines, les extraits végétaux et animaux, les silicones, les filtres solaires, et d'autres agents traitants des fibres kératiniques.

Parmi les agents propulseurs utilises de préférence dans ces compositions de mousse, on peut citer par exemple l'air comprimé, le dioxyde de carbone, le protoxyde d'azote, l'azote, l'oxyde nitreux, le diméthyléther, les hydrocarbures aliphatiques liquéfiables tels que le propane, les butanes dont l'isobutane, le pentane, l'isopentane, le néopentane, et leurs mélanges, les hydrocarbures chlorés et/ou fluorés ou leurs mélanges.

Parmi les hydrocarbures chlorés et/ou fluorés, on peut citer le monochlorodifluorométhane, le dichlorodifluorométhane, le monochlorodifluoroéthane le 1,1-difluoroéthane, et le dichlorotétrafluoroéthane, ainsi que leurs mélanges et en particulier le mélange de monochlorodifluorométhane-monochlorodifluoroéthane (40/60) ainsi que le mélange de dichlorodifluorométhane-dichlorotétrafluoroéthane (60/40).

La proportion en agent propulseur utilisée n'est pas critique mais elle détermine la densité de la mousse produite. Plus la proportion en agent propulseur est élevée, plus la densité de la mousse est faible. Généralement, les densités de mousse sont situées dans le domaine d'environ 0,02 à environ 0,20 g/cm³ et de préférence d'environ 0,05 à environ 0,15 g/cm³.

La proportion en agent propulseur est généralement comprise entre 1 et 20 % en poids par rapport au poids total de la composition aérosol, et de préférence entre 5 et 15 %.

La pression interne dans le récipient aérosol est généralement comprise entre environ 1 et 4 bars (10⁵ à 4.10⁵ Pa.).

Tout type de récipient et système de valve pour mousse aérosol est approprié pour la mise en oeuvre de l'invention selon cette forme de réalisation.

Selon un troisième mode de réalisation des compositions selon l'invention, celles-ci se présentent sous forme d'une dispersion ou d'une émulsion du type huile-dans-l'eau ou eau-dans-l'huile, et plus particulièrement sous forme d'une microémulsion ou gel.

La phase aqueuse est généralement présente en une proportion comprise entre 60 et 90 % pour les émulsions huile-dans-l'eau, et entre 30 et 60 % pour les émulsions eau-dans-l'huile, en poids par rapport au poids total de la composition.

La phase grasse est généralement présente en une proportion comprise entre 5 et 25 % pour les émulsions de type huile-dans-l'eau, et comprise entre 30 et 50 % pour les émulsions de type eau-dans-l'huile, en poids par rapport au poids total de la composition.

La phase grasse est constituée d'au moins une huile cosmétique ou d'un mélange d'au moins une huile et d'au moins une cire, celles-ci étant d'origine minérale, végétale, animale, ou synthétique, telles que décrites dans le brevet FR-2,668,927.

Parmi les agents émulsionnants susceptibles d'être utilisés dans les compositions sous forme d'émulsion huile-dans-l'eau, on peut citer entre autres le laurylsulfate, le stéarate de triéthanolamine, ou les alcools gras tels que l'alcool stéarylique ou l'alcool cétylique.

Parmi les agents émulsionnants susceptibles d'être utilisés dans les compositions sous forme d'émulsion eau-dans-l'huile, on peut citer entre autres les esters de glycérol, les alcools éthoxylés, la lanoline, l'alcool de lanoline, le cholestérol et les divers oléates de sorbitan.

Les agents émulsionnants sont généralement présents en une proportion comprise entre 1 et 10 % en poids par rapport au poids total de la composition.

Selon ce mode de réalisation, les compositions sont de préférence des microémulsions transparentes ou des gels, et contiennent un excès d'agent émulsionnant et au moins une huile minérale de faible viscosité.

Les huiles minérales susceptibles d'être utilisées dans les compositions de gels présentent des chaînes carbonées de faible longueur et sont présentes en une proportion comprise entre 15 et 20 % en poids par rapport au poids total de la composition.

Parmi les émulsionnants des compositions sous forme de gels, on peut notamment citer l'éther polyoxyéthylé et l'alcool oléylique ou son ester phosphonique, l'alcool laurylique polyéthoxylé, l'alcool oléylique polyéthoxylé, l'alcool cétylique oxyéthylé, et divers polyoxyéthylène glycols d'acides gras.

Les compositions sous forme de gels peuvent également contenir un agent de couplage tel que le 2-éthylhexane 1,3-diol, un alcool polyhydrique tel que le sorbitol, un polyéthylène glycol, la lanoline ou l'alcool de lanoline ainsi que des conservateurs.

Selon un quatrième mode de réalisation des compositions selon l'invention, celles-ci ce présentent sous forme d'une lotion.

Le véhicule de ces lotions est soit une solution aqueuse soit une solution hydroalcoolique contenant en partie sensiblement égale de l'eau déionisée et un alcool tel que l'éthanol ou l'isopropanol.

Les lotions peuvent en outre contenir au moins une substance choisie parmi un ester d'isopropyle, un polyalkylène glycol ou son oléate.

Selon un dernier mode de réalisation des compositions selon l'invention, celles-ci se présentent sous forme d'une laque et plus particulièrement d'une laque capillaire aérosol.

Le véhicule cosmétiquement acceptable généralement utilisé dans les laques est constitué d'un alcool tel que l'éthanol ou l'isopropanol ou d'un mélange hydroalcoolique.

Les laques selon ce mode particulier de réalisation contiennent en outre au moins une résine filmogène, au moins un agent plastifiant, ainsi qu'un agent propulseur.

Les résines filmogènes utilisées dans les laques sont bien connues de l'état de la technique et on peut notamment citer celles décrites dans le brevet FR-2,684,874, et elles sont de préférence présentes en une proportion de 3 à 6 % en poids par rapport au poids total de la composition.

Parmi les agents plastifiants préférés, on peut notamment citer les éthers de glycol, l'alcool benzylique, le citrate de triéthyle, le 1,3-butylèneglycol et le carbonate de propylène, présents en une proportion comprise entre X et Y% en poids par rapport au poids total de la composition.

L'agent propulseur des laques peut être choisi parmi au moins un des agents énumérés ci-dessus pour la réalisation des compositions sous forme d'une mousse.

Les laques selon ce mode de réalisation peuvent également contenir d'autres ingrédients conventionnels tels que des inhibiteurs de corrosion, des agents adoucissants, des parfums, des silicones, des filtres solaires, des colorants, des conservateurs, des agents anti-mousse, ainsi que des vitamines.

Après introduction de la composition ou "jus" dans un récipient approprié, celui-ci est muni d'un système de valve et de diffusion approprié.

Parmi les valves pouvant être utilisées, on peut notamment citer celles décrites dans le brevet FR-2,382,637.

La présente invention a également pour objet un procédé de traitement et de soins réparateurs des fibres kératiniques, désigné ci-dessus par procédé de traitement "direct", celui-ci consistant à appliquer sur les fibres kératiniques une composition telle que définie précédemment contenant au moins un dérivé de polyamino-acide et à laisser agir la composition, puis à rincer éventuellement les fibres kératiniques.

Selon un mode de réalisation particulièrement préféré du procédé de traitement et de soins réparateurs des fibres kératiniques, désigné ci-dessus par procédé de traitement "in situ", le dérivé de polyamino-acide de formule (I) est formé "in situ", c'est-à-dire directement sur les fibres kératiniques que l'on soumet au traitement, à l'aide de précurseurs donnant naissance au dérivé de polyamino-acide.

Ces précurseurs sont d'une part un N-carboxyanhydride de formule (II) suivante 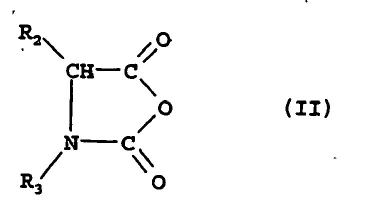 dans laquelle R₂ et R₃ sont tels que définis ci-avant, et d'autre part, un composé nucléophile de formule (III) suivante R₁-XH dans laquelle X est tel que défini ci-avant et R₁ représente:
(i) un atome d'hydrogène,
(ii) un radical alkyle, linéaire ou ramifié, saturé ou insaturé, en C₁-C₄₀, éventuellement substitué par au moins un hydroxy ou un radical 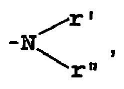 et/ou éventuellement interrompu par au moins un hétéroatome choisi parmi N, O ou Si, r' et r", identiques ou différents, étant un atome d'hydrogène ou un radical alkyle en C₁-C₆,
(iii) 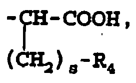 s étant 0 à 4
(iv) 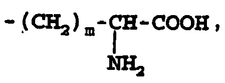 ₘ étant 3 à 5,
R₄ représentant un atome d'hydrogène, NH₂, OH, SH, -CHOHCH₃, -CONH₂, 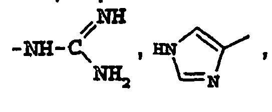 -C₆H₅ ou -C₆H₅pOH,
l'un au moins desdits précurseurs étant présent dans un milieu cosmétiquement acceptable. Pour la réalisation du traitement ces précurseurs se présentent sous forme d'un conditionnement en deux parties.

La première partie contient, sous forme solide, ou diluée dans un véhicule cosmétiquement acceptable, au moins un N-carboxyanhydride de formule (II), et la deuxième partie contient un composé nucléophile de formule (III), sous forme solide, ou diluée dans un véhicule cosmétiquement acceptable.

Le véhicule cosmétiquement acceptable du N-carboxy-anhydride de formule (II) est un solvant organique, de l'eau ou un mélange de ceux-ci. Le véhicule cosmétiquement acceptable du composé nucléophile de formule (III) est de préférence de l'eau.

Le procédé de traitement "in situ" consiste dans un premier temps à appliquer sur les cheveux, éventuellement préalablement humidifiés, la première partie du conditionnement, après dilution éventuelle à l'aide d'un milieu liquide, puis dans un deuxième temps, à appliquer la deuxième partie du conditionnement également après dilution éventuelle, notamment à base d'eau.

Selon ce procédé, on peut, si on le souhaite, réaliser préalablement le mélange des deux parties et appliquer la solution obtenue directement sur les cheveux.

On procède ensuite à la friction des cheveux, ce qui provoque, en milieu aqueux, la polycondensation des précurseurs en un dérivé de polyamino-acide. La polycondensation est généralement complète après un temps d'environ 2 à 30 minutes.

Après ce laps de temps, les cheveux sont rincés et éventuellement soumis à un shampooing.

On va maintenant donner à titre d'illustration des exemples de préparation des dérivés de polyamino-acides ainsi que des exemples de compositions cosmétiques et de procédé de traitement des cheveux.

### EXEMPLES

### Exemple 1

*Préparation du polyamino-acide de formule (I) dans laquelle* 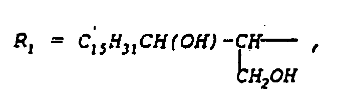 *X = -NH-, R₂ = H, R₃ = -CH₃ et n = 14,2*.

Dans un réacteur de 1 litre, équipé d'un réfrigérant, d'un thermomètre, d'une arrivée d'azote, d'une ampoule d'addition et d'un agitateur, on met en suspension 46 g (0,4 mole) de N-carboxyanhydride sarcosine dans 250 cm³ de toluène sous atmosphère d'azote. On ajoute ensuite, par petites fractions, une suspension de 8,2 g (0,027 mole) de (D/L, érythro-thréo) 2-amino ocadécane-1,3 diol dans 250 cm³ de toluène. Après la fin de l'addition, on porte le mélange réactionnel à 80°C pendant environ 3 heures. On laisse ensuite refroidir à température ambiante et on ajoute 200 cm³ d'éthanol (98°C) pour solubiliser le milieu.

Après évaporation des solvants sous pression réduite et séchage sous vide, on obtient 34,5 g d'une poudre de couleur marron.

L'indice "n" a été déterminé par RMN.

Selon le même mode operatoire que ci-dessus en faisant varier la proportion de (DL) 2-amino octadécane-1,3 diol, on a obtenu des dérivés de polyamino-acides ayant la même structure mais ayant les indices "n" suivants :
**Exemple 1(a) :** n = 9,8
**Exemple 1(b) :** n = 7,6

### Exemple 2

*Préparation du polyamino-acide de formule (I) dans laquelle* 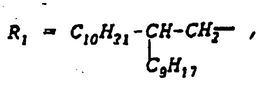 *X = -NH-, R₂ = H, R₃ = -CH₃ et n = 14*.

Dans un réacteur de 1 litre, équipé d'un réfrigérant, d'un thermomètre, d'une arrivée d'azote, d'une ampoule d'addition et d'un agitateur, on met en suspension 46 g (0,4 mole) de N-carboxyanhydride sarcosine dans 500 cm³ de toluène. On ajoute ensuite, goutte à goutte, 8,1 g (0,027 mole) de 2-octyl dodécylamine. Après la fin de l'addition, on porte le mélange à 80°C pendant environ 2 heures. On laisse ensuite refroidir à température ambiante puis ajoute 50 cm³ d'éthanol (95°C). Après évaporation des solvants sous pression réduite et séchage sous vide, on obtient 36,7 g d'une poudre de couleur marron.

L'indice "n" a été déterminé par RMN.

Selon le même mode opératoire que ci-dessus, mais en faisant varier la proportion de 2-octyl dodécylamine, on a obtenu des dérivés de polyamino-acides ayant la même structure mais ayant les indices "n" suivants :
**Exemple 2(a) :** n = 9,6
**Exemple 2(b) :** n = 7,4

### Exemple 3

*Préparation du polyamino-acide de formule (I) dans laquelle R₁ = C₁₆H₃₃, X = -NN-, R₂ = H, R₃ = -CH₃ et n = 7,2*.

Ce polyamino-acide est obtenu selon le même mode opératoire que celui décrit à l'exemple 2 mais en faisant réagir 12 g (0,05 mole) d'hexadécylamine avec la N-carboxyanhydride sarcosine.

Après évaporation des solvants et séchage sous vide, on obtient 40g d'une poudre.

En faisant varier la proportion d'hexadécylamine, on a obtenu les dérivés de polyamino-acides ayant la même structure mais ayant la même structure mais ayant les indices "n" suivants :
**Exemple 3(a)** : n = 9, 2
**Exemple 3(b)** : n = 12,5

### Exemple 4

*Préparation d'un dérivé de polyamino-acide de formule (I) dans laquelle R₁ = C₈H₁₇-CH=CH-C₈H₁₆-, X = -NH-, R₂ = H, R₃ = -CH₃ et n = 7,4*.

Ce polyamino-acide est obtenu selon le même mode opératoire que celui décrit à l'exemple 2 mais en faisant réagir 13 g (0,05 mole) d'oléylamine avec la N-carboxyanhydride sarcosine.

Après évaporation des solvants et séchage sous vide, on obtient 42g d'une poudre.

En faisant varier la proportion d'oléylamine, on a obtenu les dérivés de polyamino-acides ayant la même structure mais ayant la même structure mais ayant les indices "n" suivants :
**Exemple 4(a) :** n = 10, 5
**Exemple 4(b) :** n = 13, 2

### Exemple 5

*Préparation du polyamino-acide de formule (I) dans laquelle* 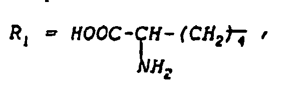 *X = -NH-, R₂ = H, R₃ = -CH₃ et n = 12,1*.

Dans un erlenmeyer de 500 mL sous agitation, on introduit 10 g (0,08 mole) de N-carboxyanhydride sarcosine puis ajoute 100 cm³ d'eau distillée (pH 6,7) et en une seule fois 1 g (0,006 mole) de lysine.

Un dégagement important de CO₂ se produit et l'on poursuit l'agitation du mélange pendant environ 30 minutes à température ambiante. Après évaporation de l'eau sous pression réduite et séchage sous vide, on obtient 6,7 g d'une poudre de couleur brune.

L'indice "n" a été déterminé par RMN.

Selon le même mode opératoire que ci-dessus, mais en faisant varier la proportion de lysine, on a obtenu des dérivés de polyamino-acides ayant la même structure mais ayant les indices "n" théoriques suivants :
**Exemple 5(a)** : n = 50
**Exemple 5(b)** : n = 25
**Exemple 5(c)** : n = 6
**Exemple 5(d)** : n = 3

### Exemple 6

*Préparation du polyamino-acide de formule (I) dans laquelle* 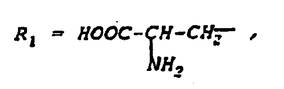 *X = S, R₂ = H, R₃ = -CH₃*
*et n = 13 (indice théorique).*

Ce polyamino-acide est obtenu selon le même mode opératoire que celui décrit à l'exemple 5 mais en remplaçant la lysine par la quantité molaire correspondante de cystéine.

### Exemple 7

*Préparation du polyamino-acide de formule (I) dans laquelle* 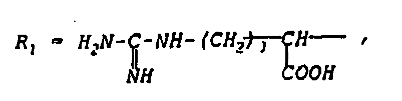 *X = -NH-, R₂ = H, R₃ = -CH₃*
*et n = 12 (indice théorique)*.

Ce polyamino-acide est obtenu selon le même mode opératoire que celui décrit à l'exemple 5 mais en remplaçant la lysine par la quantité molaire correspondante d'arginine.

### Exemple 8

*Préparation du polyamino-acide de formule (I) dans laquelle* 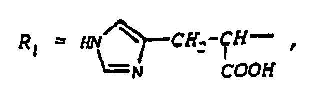 *X* = *-NH-, R₂* = *H, R₃ = -CH₃*
et *n* = 12 *(indice théorique).*

Ce polyamino-acide est obtenu selon le même mode opératoire que celui décrit à l'exemple 5 mais en remplaçant la lysine par la quantité molaire correspondante d'histidine.

### Exemple 9

*Préparation du polyamino-acide de formule (I) dans laquelle* 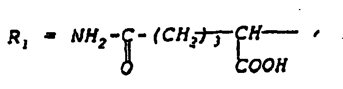 *X* = *-NH-, R₂* = *H, R₃ = -CH₃*
*et n = 13 (indice théorique).*

Ce polyamino-acide est obtenu selon le même mode opératoire que celui décrit à l'exemple 5 mais en remplaçant la lysine par la quantité molaire correspondante de glutamine.

### Exemple 10

*Préparation du polyamino-acide de formule (I) dans laquelle* 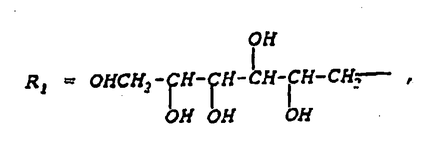 *X* = *-NH-, R₂ = H, R₃ = -CH₃*
*et n =12 (indice théorique)*.

Ce polyamino-acide est obtenu selon le même mode opératoire que celui décrit à l'exemple 5 mais en remplaçant la lysine par la quantité molaire correspondante de glucamine.

### EXEMPLES DE COMPOSITION COSMETIQUE

On a préparé les formulations suivantes pour le soin et le traitement des cheveux :

### Exemple 11 : Shampooing

- Lauryl éther sulfate de sodium (28 % M.A.) 75 g
- Monoisopropanolamide d'acide de coprah vendu par la Société Albright et Wilson sous la dénomination "Empilan CIS®" 1 g
- Polyamino-acide de l'exemple 5 1 g
- Eau q.s.p. 100 g

Le shampooing ainsi formulé est d'aspect limpide.

### Exemple 12 : Soin après-shampooing à rincer

- Méthosulfate de 1-méthyl 2-suif 3-sulfamido éthylimidazolium/propène glycol (75/25) vendu par la Société Witco sous la dénomination "REWOQUAT W75PG®" 2 g M.A
- Polyamino-acide de l'exemple 5 0,5 g
- Mélange d'alcool cétylique et cétyl stéarylique oxyéthyléné 3 g
- Conservateur, parfum q.s.
- pH spontané de 5,2
- Eau q.s.p. 100 g

### Exemple 13 : Shampooing

- Lauryl éther sulfate de sodium (28 % M.A.) 60 g
- Cocoyl bétaine 9 g
- Polyamino-acide de l'exemple 1 0,5 g
- Conservateur, parfum q.s.
- HCl q.s. pH 6
- Eau q.s.p. 100 g

Le shampooing ainsi formulé est opalescent.

### Exemple 14 : Lotion démêlante à rincer

- Chlorure de béhényltriméthylammonium à 80 % dans un mélange eau/isopropanol (15/85) vendu par la Société Toho sous la dénomination "CATINAL DC 50®" 0,5 g M.A.
- Polyamino-acide de l'exemple 4. 0,1 g
- Conservateur, parfum q.s.
- NaOH q.s. pH 5,5
- Eau q.s.p. 100 g

On a réalisé les exemples suivants de traitement renforçateur sur tête ("in situ").

### Exemple 15 : Procédé de traitement des cheveux à partir d'un conditionnement en deux parties

On applique tout d'abord sur des cheveux préalablement mouillés, un premier conditionnement contenant une solution de N-carboxyanhydride sarcosine diluée à 10 % soit dans du SOKETAL® (D,L-α,β isopropylidène glycérol), soit dans du diméthyl éther isosorbide. La solution ainsi appliquée doit imprégner la totalité de la chevelure. Puis on applique ensuite un deuxième conditionnement contenant une solution de L-lysine diluée à 4 % dans de l'eau. On laisse agir pendant 15 minutes puis les cheveux sont rincés à l'eau tiède, soumis à un shampooing et, enfin, séchés.

### Exemple 16 : Procédé de traitement des cheveux à partir d'un conditionnement solide en deux parties

De la N-carboxyanhydride sarcosine conditionnée sous forme solide est tout d'abord appliquée sur une chevelure préalablement mouillée qui est ensuite massée pendant environ 5 minutes. Puis une solution de L-lysine diluée à 4 % dans l'eau, conditionnée séparément, est ajoutée ensuite sur la chevelure.

On laisse alors agir pendant environ 15 minutes, puis les cheveux sont rincés à l'eau tiède, lavés à l'aide d'un shampooing et séchés.

Les cheveux traités selon l'exemple 15 ou 16 présentent une visible amélioration de tenue et de volume.

### ESSAIS COMPARATIFS

### Etude des propriétés de renforcement des fibres kératiniques

Les propriétés de renforcement des cheveux naturels et décolorés ont été évaluées après traitement selon le procédé "in situ".

Des mèches de 0,6 g et de 15 cm d'une part de cheveux naturels châtain foncé et, d'autre part, de cheveux décolorés ont tout d'abord été immergées dans un tampon phosphate à 37°C, puis rincées à l'eau, puis soumises à un shampooing, et à nouveau rincées.

Une partie des mèches obtenues sert de témoin et les autres ont ensuite été traitées à l'aide d'une solution réalisée au moment de l'emploi en mélangeant 1g de N-carboxyanhydride-sarcosine (NCA-sarcosine) et 0,1 g de lysine dans un tampon phosphate à 37°C. Après une période d'environ 30 minutes au cours de laquelle se déroule la réaction de polycondensation, les mèches ont ensuite été rincées à l'eau, puis soumises à un shampooing, et à nouveau rincées.

Le renforcement des mèches ainsi traitées a été quantifié à l'aide du test du pendule de souplesse. Ce test du pendule de souplesse consiste à mesurer le nombre d'oscillations d'un pendule ayant un poids de lest de 47 g qui vient courber un échantillon de 39 cheveux de 10 mm de longueur. Les caractéristiques de renforcement des cheveux sont inversement proportionnelles au nombre d'oscillations du pendule. En effet, toute augmentation du renforcement des cheveux se traduit par une diminution du nombre d'oscillations du pendule.

Les mesures du nombre d'oscillations sur trois échantillons de mèches de cheveux naturels et décolorés, à une température de 25°C, et à une humidité relative de 45 %, sont données dans le Tableau 1 ci-dessous :

**Tableau 1**

| Nombre d'oscillations | Cheveux naturels | Erreur-type | Cheveux décolorés | Erreur-type |
|---|---|---|---|---|
| Témoin tampon phosphate | 159 | 2 | 130 | 2 |
| NCA-sarcosine/lysine *(essai 1)* | 148 | 2 | 125 | 1 |
| NCA-sarcosine/lysine *(essai 2)* | 149 | 2 | 130 | 1 |
| NCA-sarcosine/lysine *(essai 3)* | 150 | 1 | 125 | 1 |
| Variation en % | -6 | p=0,0001 | -3 | p=0,04 |

La différence observée du nombre d'oscillations entre les cheveux naturels et les cheveux décolorés, avant tout traitement, s'explique essentiellement par le fait que la décoloration provoque une augmentation importante de la rigidité des cheveux.

Les résultats du Tableau 1 ci-dessus montrent une diminution reproductible du nombre d'oscillations du pendule, de moins de 6% pour les cheveux naturels, et de moins de 3% pour les cheveux décolorés.

Le traitement "in situ" confère donc, après la réaction de polycondensation, une augmentation significative des propriétés intrinsèques de rigidité des cheveux naturels et décolorés. Ces caractéristiques sont conservées et toujours visibles même après avoir soumis les mèches à un shampooing puis à un rinçage.

### Quantification du dépôt de polylysine-sarcosine sur les fibres kératiniques

On a déterminé le dépôt de polylysine-sarcosine par dosage de la quantité de sarcosine résiduelle à la surface de mèches de cheveux naturels ou décolorés après traitement "direct" et après traitement "in situ".

La quantification du dépôt de sarcosine a été réalisée après rinçage des mèches traitées, et après rincage et shampooing des mèches traitées.

Les mèches de cheveux après ces opérations ont été soumises à une hydrolyse acide, et la quantité de sarcosine résiduelle a été mesurée à l'aide de l'autoanalyseur d'acides aminés HITACHI L8500A.

Les quantités de sarcosine retrouvées à la surface des échantillons de cheveux naturels et colorés sont données dans le Tableau 2 ci-dessous. Ces quantités sont exprimées en grammes de sarcosine présents sur le cheveu par 100 grammes de cheveu.

**Tableau 2**

| | TRAITEMENTS | | | | |
|---|---|---|---|---|---|
| | Traitement "in situ" | Traitement "direct" | Après rinçage | Après shampooing | en g de sarcosine pour 100 g de cheveux |
| *Témoin* | | | + | | 0 |
| CHEVEUX NATURELS | + | | + | + | 0,26 |
| | + | | + | | 0,32 |
| | | + | + | + | 0,16 |
| | | + | + | | 0,25 |
| *Témoin* | | | + | | 0 |
| CHEVEUX DECOLORES | + | | + | + | 0,38 |
| | + | | + | | 0,67 |
| | | + | + | + | 0,36 |
| | | + | + | | 0,55 |

D'après le Tableau 2 ci-dessus, on peut constater qu'il y a un dépôt notable de sarcosine après traitement et rinçage. Ce dépôt de sarcosine demeure par ailleurs en une proportion satisfaisante après un shampooing ultérieur.

## Revendications

1. Utilisation pour le renforcement et le soin des fibres kératiniques, d'au moins un dérivé de polyamino-acide de formule générale (I) suivante : 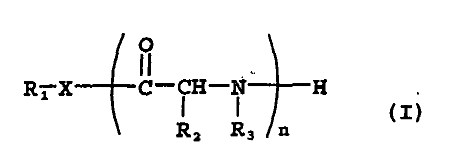 dans laquelle :
- X est -O-, -S- ou -NR₃
- R₁ représente:
(ii) un radical alkyle, linéaire ou ramifié, saturé ou insaturé, en C₁-C₄₀, éventuellement substitué par au moins un hydroxy ou un radical 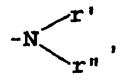 et/ou éventuellement interrompu par au moins un hétéroatome choisi parmi N, O ou Si, r' et r", identiques ou différents, étant un atome d'hydrogène ou un radical alkyle en C₁-C₆,
(iii) 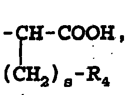 s étant 0 à 4
(iv) 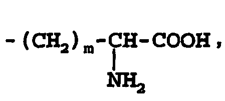 ₘ étant 3 à 5,
- R₂ représente un atome d'hydrogène, un radical alkyle en C₁-C₈, -CH₂C₆H₅, - CH₂C₆H₅pOH, -CH₂OH, -CHOH-CH₃ ou -(CH₂)ₜ-NH₂, ₜ étant 3 à 5,
- R₃ représente un atome d'hydrogène ou un radical alkyle en C₁-C₆,
- R₄ représente un atome d'hydrogène, NH₂, OH, SH, -CHOHCH₃, -CONH₂, 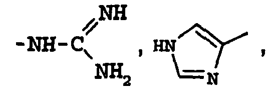 -C₆H₅ ou -C₆H₅pOH, et
- n est un nombre moyen supérieur à 1 tel que le poids moléculaire du dérivé de polyamino-acide soit compris entre 200 et 200 000, l'unité répétitive étant soit identique pour un même dérivé, soit différente, R₂ et/ou R₃ prenant alors au moins l'une des autres significations données pour ces radicaux,
ou d'un sel dudit dérivé de polyamino-acide, dans une composition cosmétique.

2. Utilisation selon la revendication 1, **caractérisée par le fait que** la composition contient le dérivé de polyamino-acide en une proportion comprise entre 0,001 et 30 % en poids par rapport au poids total de la composition.

3. Utilisation selon l'une quelconque des revendications précédentes, **caractérisée par le fait que** la composition contient le dérivé de polyamino-acide en une proportion comprise entre 0,01 et 15 % en poids par rapport au poids total de la composition.

4. Utilisation selon l'une quelconque des revendications précédentes, **caractérisée par le fait que** la composition cosmétique se présente sous forme d'un shampooing, le véhicule cosmétique étant choisi parmi une solution aqueuse et une solution hydroalcoolique.

5. Utilisation selon la revendication 4, **caractérisée par le fait que** la composition sous forme d'un shampooing comprend en outre un tensioactif présent en une proportion comprise entre 0,01 et 50 % en poids par rapport au poids total de la composition.

6. Utilisation selon les revendications 1 à 3, **caractérisée par le fait que** la composition cosmétique se présente sous forme d'une mousse, le véhicule cosmétique étant choisi parmi une phase grasse et un mélange hydroalcoolique.

7. Utilisation selon la revendication 6 **caractérisée par le fait que** la composition sous forme d'une mousse comprend en outre au moins un agent moussant et au moins un agent propulseur.

8. Utilisation selon la revendication 7, **caractérisée par le fait que** l'agent moussant est présent en une proportion comprise entre 0,05 et 20 % en poids par rapport au poids total de la composition.

9. Utilisation selon la revendication 8, **caractérisée par le fait que** l'agent propulseur est présent en une proportion comprise entre 1 et 20 % en poids par rapport au poids total de la composition.

10. Utilisation selon les revendications 1 à 3, **caractérisée par le fait que** la composition cosmétique se présente sous forme d'une émulsion eau-dans-l'huile dont la phase grasse est présente en une proportion comprise entre 30 et 50% en poids par rapport au poids total de la composition.

11. Utilisation selon les revendications 1 à 3, **caractérisée par le fait que** la composition cosmétique se présente sous forme d'une émulsion huile-dans-l'eau dont la phase grasse est présente en une proportion comprise entre 5 à 25% en poids par rapport au poids total de la composition.

12. Utilisation selon les revendications 10 et 11, **caractérisée par le fait que** la composition sous forme d'une émulsion contient en outre un agent émulsionnant présent en une proportion comprise entre 1 et 10% en poids par rapport au poids total de la composition.

13. Utilisation selon la revendication 11, **caractérisée par le fait que** l'émulsion huile-dans-l'eau se présente sous forme d'une microémulsion ou gel, ladite phase grasse comprenant au moins une huile minérale de faible viscosité.

14. Utilisation selon la revendication 13, **caractérisée par le fait que** l'huile minérale est présente en une proportion comprise entre 15 et 20% en poids par rapport au poids total de la composition de gel.

15. Utilisation selon les revendications 1 à 3, **caractérisée par le fait que** la composition cosmétique se présente sous forme d'une lotion, le véhicule cosmétique étant une solution aqueuse ou hydroalcoolique.

16. Utilisation selon les revendications 1 à 3, **caractérisée par le fait que** la composition cosmétique se présente sous forme d'une laque, le véhicule cosmétique étant un alcool ou un mélange hydroalcoolique.

17. Utilisation selon la revendication 16, **caractérisée par le fait que** la composition de laque comprend en outre une résine filmogène, au moins un agent plastifiant et au moins un agent propulseur.

18. Utilisation selon la revendication 17, **caractérisée par le fait que** la résine filmogène est présente en une proportion comprise entre 3 et 6% en poids par rapport au poids total de la composition.

19. Utilisation selon l'une quelconque des revendications précédentes, **caractérisée par le fait que** la composition cosmétique contient en outre au moins un ingrédient conventionnel choisi parmi les agents adoucissants, les parfums, les extraits végétaux et animaux, les céramides, les silicones, les filtres solaires, les colorants, les agents antimicrobiens, les vitamines, les conservateurs, les agents séquestrants, les agents régulateurs de pH et d'autres substances actives traitantes des fibres kératiniques.

20. Procédé de traitement des fibres kératiniques **caractérisé par le fait qu'**il consiste à appliquer, simultanément ou séparément, sur lesdites fibres kératiniques éventuellement humidifiées, des précurseurs d'un polyamino-acide (a) et (b) suivants :
(a) un N-carboxyanhydride de formule (II) suivante : 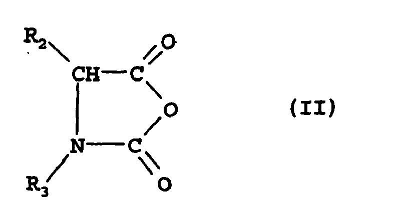 dans laquelle R₂ et R₃ sont tels que définis à la revendication 1, et
(b) un composé nucléophile de formule (III) suivante : R₁-XH dans laquelle X est tel que défini dans la revendication 1 et R₁ représente :
(i) un atome d'hydrogène,
(ii) un radical alkyle, linéaire ou ramifié, saturé ou insaturé, en C₁-C₄₀, éventuellement substitué par au moins un hydroxy ou un radical 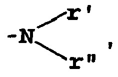 et/ou éventuellement interrompu par au moins un hétéroatome choisi parmi N, O ou Si, r' et r", identiques ou différents, étant un atome d'hydrogène ou un radical alkyle en C₁-C₆,
(iii) 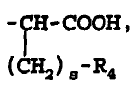 s étant 0 à 4
(iv) 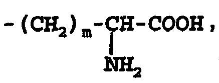 ₘ étant 3 à 5,
R₄ représentant un atome d'hydrogène, NH₂, OH, SH, -CHOHCH₃, -CONH₂, 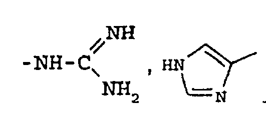 -C₆H₅ ou -C₆H₅pOH,
l'un au moins desdits précurseurs étant présent dans un milieu cosmétiquement acceptable,
à frictionner lesdites fibres pendant un temps d'environ 2 à 30 minutes, et à éventuellement rincer.

21. Procédé de traitement des fibres kératiniques selon la revendication 20, **caractérisé par le fait qu'**après l'étape de rinçage, les fibres kératiniques sont soumises à un shampooing.

22. Conditionnement en deux parties pour la mise en oeuvre du procédé selon les revendications 20 et 21, **caractérisé par le fait que** la première partie contient sous forme solide ou diluée dans un véhicule cosmétiquement acceptable au moins un N-carboxyanhydride de formule (II) telle que définie à la revendication 20 et que la deuxième partie contient sous forme solide ou diluée dans un véhicule cosmétiquement acceptable au moins un composé nucléophile de formule (III) telle que définie à la revendication 20.

23. Conditionnement en deux parties selon la revendication 22, **caractérisé par le fait que** le véhicule cosmétiquement acceptable du N-carboxyanhydride de formule (II) est un solvant organique, de l'eau ou un mélange de ceux-ci.

24. Conditionnement en deux parties selon la revendication 22, **caractérisé par le fait que** le véhicule cosmétiquement acceptable du composé nucléophile de formule (III) est de l'eau.

25. Conditionnement en deux parties selon l'une quelconque des revendications 22 à 24, **caractérisé par le fait qu'**au moins une des deux parties contient au moins un ingrédient cosmétique conventionnel tel que défini selon la revendication 19.

26. Utilisation, dans une composition cosmétique capillaire, d'au moins un dérivé de polyamino-acide de formule générale (I) telle que définie à la revendication 1, pour améliorer la tenue et le volume des cheveux.

## Claims

1. Use, for strengthening and caring for keratin fibres, of at least one polyamino acid derivative of general formula (I) below: 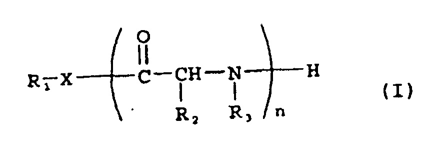 in which:
X is -O-, -S- or -NR₃-,
R₁ represents :
(ii) a linear or branched, saturated or unsaturated, C₁-C₄₀ alkyl radical, optionally substituted with at least one hydroxyl or with a radical 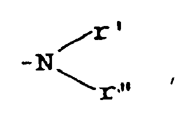 and/or optionally interrupted with at least one hetero atom chosen from N, O or Si, r' and r'', which may be identical or different, being a hydrogen atom or a C₁-C₆ alkyl radical,
(iii) 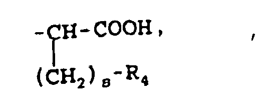 s being 0 to 4,
(iv) 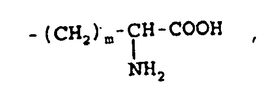 m being 3 to 5,
R₂ represents a hydrogen atom or a C₁-C₈ alkyl -CH₂C₆H₅, -CH₂C₆H₅pOH, -CH₂OH, -CHOH-CH₃ or -(CH₂)ₜ-NH₂ radical, t being 3 to 5,
R₃ represents a hydrogen atom or a C₁-C₆ alkyl radical,
R₄ represents a hydrogen atom, NH₂, OH, SH, -CHOHCH₃, CONH₂, 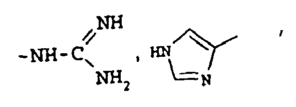 -C₆H₅ or -C₆H₅pOH, and
n is an average number greater than 1 such that the molecular weight of the polyamino acid derivative is between 200 and 200 000, the repeating unit being either identical for the same derivative, or different, R₂ and/or R₃ then taking at least one of the other meanings given for these radicals,
or of a salt of the said polyamino acid derivative, in a cosmetic composition.

2. Use according to Claim 1, **characterized in that** the composition contains the polyamino acid derivative in a proportion of between 0.001% and 30% by weight relative to the total weight of the composition.

3. Use according to either of the preceding claims **characterized in that** the composition contains the polyamino acid derivative in a proportion of between 0.01% and 15% by weight relative to the total weight of the composition.

4. Use according to any one of the preceding claims, **characterized in that** the cosmetic composition is in the form of a shampoo, the cosmetic vehicle being chosen from an aqueous solution and an aqueous-alcoholic solution.

5. Use according to Claim 4, **characterized in that** the composition in the form of a shampoo also comprises a surfactant present in a proportion of between 0.01% and 50% by weight relative to the total weight of the composition.

6. Use according to Claims 1 to 3, **characterized in that** the cosmetic composition is in the form of a mousse, the cosmetic vehicle being chosen from a fatty phase and an aqueous-alcoholic mixture.

7. Use according to Claim 6, **characterized in that** the composition in the form of a mousse also comprises at least one foaming agent and at least one propellant.

8. Use according to Claim 7, **characterized in that** the foaming agent is present in a proportion of between 0.05% and 20% by weight relative to the total weight of the composition.

9. Use according to Claim 8, **characterized in that** the propellant is present in a proportion of between 1% and 20% by weight relative to the total weight of the composition.

10. Use according to Claims 1 to 3, **characterized in that** the cosmetic composition is in the form of a water-in-oil emulsion whose fatty phase is present in a proportion of between 30% and 50% by weight relative to the total weight of the composition.

11. Use according to Claims 1 to 3, **characterized in that** the cosmetic composition is in the form of an oil-in-water emulsion whose fatty phase is present in a proportion of between 5% and 25% by weight relative to the total weight of the composition.

12. Use according to Claims 10 and 11, **characterized in that** the composition in the form of an emulsion also contains an emulsifier present in a proportion of between 1% and 10% by weight relative to the total weight of the composition.

13. Use according to Claim 11, **characterized in that** the oil-in-water emulsion is present in the form of a microemulsion or gel, the said fatty phase comprising at least one mineral oil of low viscosity.

14. Use according to Claim 13, **characterized in that** the mineral oil is present in a proportion of between 15% and 20% by weight relative to the total weight of the gel composition.

15. Use according to Claims 1 to 3, **characterized in that** the cosmetic composition is in the form of a lotion, the cosmetic vehicle being an aqueous or aqueous-alcoholic solution.

16. Use according to Claims 1 to 3, **characterized in that** the cosmetic composition is in the form of a lacquer, the cosmetic vehicle being an alcohol or an aqueous-alcoholic mixture.

17. Use according to Claim 16, **characterized in that** the lacquer composition also comprises a film-forming resin, at least one plasticizer and at least one propellant.

18. Use according to Claim 17, **characterized in that** the film-forming resin is present in a proportion of between 3% and 6% by weight relative to the total weight of the composition.

19. Use according to any one of the preceding claims, **characterized in that** the cosmetic composition also contains at least one conventional ingredient chosen from softeners, fragrances, plant and animal extracts, ceramides, silicones, sunscreens, dyes, antimicrobial agents, vitamins, preserving agents, sequestering agents, pH regulators and other active substances for treating keratin fibres.

20. Process for treating keratin fibres, **characterized in that** it consists in applying, simultaneously or separately, to the said keratin fibres which have optionally been moistened, precursors of a polyamino acid (a) and (b) below:
(a) an N-carboxyanhydride of formula (II) below: 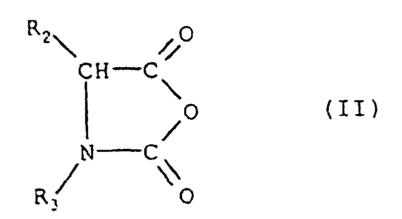 in which R₂ and R₃ are as defined in Claim 1, and
(b) a nucleophilic compound of the following formula (III): R₁-XH, in which X is as defined in Claim 1 and R1 represents:
(i) a hydrogen atom,
(ii) a linear or branched, saturated or unsaturated, C₁-C₄₀ alkyl radical, optionally substituted with at least one hydroxyl or with a radical 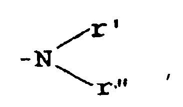 and/or optionally interrupted with at least one hetero atom chosen from N, O or Si, r' and r'', which may be identical or different, being a hydrogen atom or a C₁-C₆ alkyl radical,
(iii) 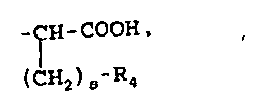 s being 0 to 4,
(iv) 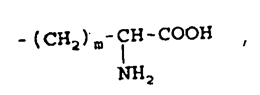 m being 3 to 5,
R₄ representing a hydrogen atom, NH₂, OH, SH, -CHOHCH₃, CONH₂, 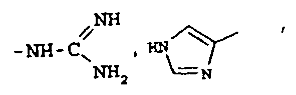 -C₆H₅ or -C₆H₅pOH, at least one of the said precursors being present in a cosmetically acceptable medium,
in rubbing the said fibres for a period of about 2 to 30 minutes and in optionally rinsing.

21. Process for treating keratin fibres according to Claim 20, **characterized in that,** after the rinsing step, the keratin fibres are shampooed.

22. Packaging in two parts for carrying out the process according to Claims 20 and 21, **characterized in that** the first part contains, in solid form or diluted in a cosmetically acceptable vehicle, at least one N-carboxyanhydride of formula (II) as defined in Claim 20, and **in that** the second part contains, in solid form or diluted in a cosmetically acceptable vehicle, at least one nucleophilic compound of formula (III) as defined in Claim 20.

23. Packaging in two parts according to Claim 22, **characterized in that** the cosmetically acceptable vehicle for the N-carboxyanhydride of formula (II) is an organic solvent, water or a mixture thereof.

24. Packaging in two parts according to Claim 22, **characterized in that** the cosmetically acceptable vehicle for the nucleophilic compound of formula (III) is water.

25. Packaging in two parts according to any one of Claims 22 to 24, **characterized in that** at least one of the two parts contains at least one conventional cosmetic ingredient as defined according to Claim 19.

26. Use, in a cosmetic hair composition, of at least one polyamino-acid derivative of general formula (I) as defined in Claim 1, to improve the hold and volume of the hair.

## Patentansprüche

1. Verwendung, zur Kräftigung und Pflege von Keratinfasern, wenigstens eines Polyaminosäurederivats der folgenden allgemeinen Formel (I): 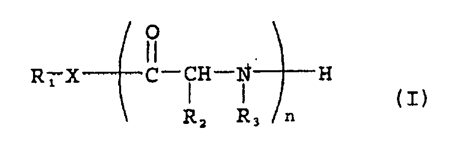 wobei:
- X für -O-, -S- oder -NR₃ steht,
- R₁ folgendes repräsentiert:
(ii) ein C₁-C₄₀ Alkylradikal, linear oder verzweigt, gesättigt oder ungesättigt, möglicherweise durch wenigstens ein Hydroxid oder ein Radikal 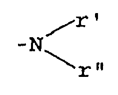 substituiert, und/oder möglicherweise durch wenigstens ein Heteroatom unterbrochen ist, das unter N, O oder Si ausgewählt ist, wobei r' und r", ob identisch oder verschieden, ein Wasserstoffatom oder ein C₁-C₆ Alkylradikal sind,
(iii) 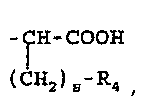 mit s 0 bis 4 ist,
(iv) 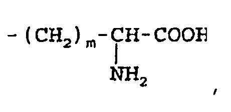 mit m 3 bis 5 ist,
- R₂ ein Wasserstoffatom, ein C₁-C₈ Alkylradikal, -CH₂C₆H₅, -CH₂C₆H₅pOH, -CH₂OH, -CHOH-CH₃ oder -(CH₂)ₜ-NH₂ repräsentiert, mit t 3 bis 5 ist,
- R₃ ein Wasserstoffatom oder ein C₁-C₆ Alkylradikal repräsentiert,
- R₄ ein Wasserstoffatom, NH₂, OH, SH, -CHOHCH₃, -CONH₂, 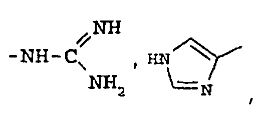 -C₆H₅ oder -C₆H₅pOH repräsentiert, und
- n eine durchschnittliche Zahl größer als 1 ist, so dass das Molekulargewicht des Polyaminosäurederivats zwischen 200 und 200,000 liegt, wobei die Wiederholungseinheit für ein nämliches Derivat identisch ist oder unterschiedlich sein kann, R₂ und/oder R₃ dann wenigstens eine der anderen Bedeutungen annimmt, die für diese Radikale vorgegeben sind,
oder ein Salz des Polyaminosäurederivats, in einer kosmetischen Zusammensetzung.

2. Verwendung nach Anspruch 1, **dadurch gekennzeichnet, dass** die Zusammensetzung das Polyaminosäurederivat in einem Verhältnis zwischen 0,001 und 30 Gew. -% enthält, bezogen auf das Gesamtgewicht der Zusammensetzung.

3. Verwendung nach einem der vorhergehenden Ansprüche, **dadurch gekennzeichnet, dass** die Zusammensetzung das Polyaminosäurederivat in einem Verhältnis zwischen 0,01 und 15 Gew.-% enthält, bezogen auf das Gesamtgewicht der Zusammensetzung.

4. Verwendung nach einem der vorhergehenden Ansprüche, **dadurch gekennzeichnet, dass** die kosmetische Zusammensetzung in Form eines Schampons erzeugt wird, wobei der kosmetische Träger unter einer wässerigen Lösung und einer hydroalkoholischen Lösung ausgewählt ist.

5. Verwendung nach Anspruch 4, **dadurch gekennzeichnet, dass** die in Form eines Schampons erzeugte Zusammensetzung außerdem einen grenzflächenaktiven Stoff enthält, in einem Verhältnis zwischen 0,01 und 50 Gew.-% bezogen auf das Gesamtgewicht der Zusammensetzung.

6. Verwendung nach den Ansprüchen 1 bis 3, **dadurch gekennzeichnet, dass** die kosmetische Zusammensetzung in Form eines Schaums erzeugt wird, wobei der kosmetische Träger unter einer fettigen Phase und einem hydroalkoholischen Gemisch ausgewählt ist.

7. Verwendung nach Anspruch 6, **dadurch gekennzeichnet, dass** die in Form eines Schaums erzeugte Zusammensetzung außerdem wenigstens eine schäumende Substanz und wenigstens eine Treibgassubstanz enthält.

8. Verwendung nach Anspruch 7, **dadurch gekennzeichnet, dass** die schäumende Substanz in einem Verhältnis im Bereich von 0,05 und 20 Gew.-%, bezogen auf das Gesamtgewicht der Zusammensetzung, enthalten ist.

9. Verwendung nach Anspruch 8, **dadurch gekennzeichnet, dass** die Treibgassubstanz in einem Verhältnis zwischen 1 und 20 Gew.-%, bezogen auf das Gesamtgewicht der Zusammensetzung, enthalten ist.

10. Verwendung nach den Ansprüchen 1 bis 3, **dadurch gekennzeichnet, dass** die kosmetische Zusammensetzung in Form einer Wasser-in-Öl-Emulsion erzeugt ist, deren fettige Phase in einem Verhältnis zwischen 30 und 50 Gew.-%, bezogen auf das Gesamtgewicht der Zusammensetzung, enthalten ist.

11. Verwendung nach den Ansprüchen 1 bis 3, **dadurch gekennzeichnet, dass** die kosmetische Zusammensetzung in Form einer Öl-in-Wasser-Emulsion erzeugt ist, deren fettige Phase in einem Verhältnis zwischen 5 zu 25 Gew.-%, bezogen auf das Gesamtgewicht der Zusammensetzung, enthalten ist.

12. Verwendung nach den Ansprüchen 10 und 11, **dadurch gekennzeichnet, dass** die in Form einer Emulsion erzeugte Zusammensetzung außerdem eine Emulgatorsubstanz enthält, die in einem Verhältnis zwischen 1 und 10 Gew.-%, bezogen auf das Gesamtgewicht der Zusammensetzung, enthalten ist.

13. Verwendung nach Anspruch 11, **dadurch gekennzeichnet, dass** die Öl-in-Wasser-Emulsion in Form einer Mikroemulsion oder eines Gels erzeugt ist, wobei die fettige Phase mindestens ein Mineralöl geringer Viskosität enthält.

14. Verwendung nach Anspruch 13, **dadurch gekennzeichnet, dass** das Mineralöl in einem Verhältnis zwischen 15 und 20 Gew. -%, bezogen auf das Gesamtgewicht der Gelzusammensetzung, enthalten ist.

15. Verwendung nach den Ansprüchen 1 bis 3, **dadurch gekennzeichnet, dass** die kosmetische Zusammensetzung in Form einer Lotion erzeugt ist, wobei der kosmetische Träger eine wässrige oder hydroalkoholische Lösung ist.

16. Verwendung nach den Ansprüchen 1 bis 3, **dadurch gekennzeichnet, dass** die kosmetische Zusammensetzung in Form eines Lacks erzeugt ist, wobei der kosmetische Träger ein Alkohol oder ein hydroalkoholisches Gemisch ist.

17. Verwendung nach Anspruch 16, **dadurch gekennzeichnet, dass** die Lackzusammensetzung außerdem ein filmbildendes Harz, wenigstens einen Weichmacher und wenigstens eine Treibgassubstanz enthält.

18. Verwendung nach Anspruch 17, **dadurch gekennzeichnet, dass** das filmbildende Harz in einem Verhältnis zwischen 3 und 6 Gew.-%, bezogen auf das Gesamtgewicht der Zusammensetzung, anwesend ist.

19. Verwendung nach einem der vorhergehenden Ansprüche, **dadurch gekennzeichnet, dass** die kosmetische Zusammensetzung außerdem wenigstens einen herkömmlichen Bestandteil enthält, der aus der Gruppe ausgewählt ist, zu der gehören: Weichmacher, Parfums, pflanzliche und tierische Extrakte, Ceramide, Silikone, UV-Filter, Farbstoffe, Antimikrobiotika, Vitamine, Konservierungsstoffe, Sequestrierstoffe, pH-Wert-Regulatoren und sonstige aktive Substanzen zur Behandlung von Keratinfasern.

20. Verfahren zur Behandlung von Keratinfasern **dadurch gekennzeichnet, dass** zu dem Verfahren die Schritte gehören: gleichzeitiges oder gesondertes Auftragen folgender Precursorstoffe einer Polyaminosäure (a) und (b) auf die möglicherweise befeuchteten Keratinfasern:
(a) ein N-Carboxyanhydrid der folgenden Formel (II): 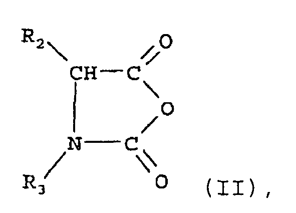 wobei R₂ und R₃ wie in Anspruch 1 definiert sind, und
(b) eine nucleophile Verbindung der folgenden Formel (III): R₁-XH ist, wobei X wie in Anspruch 1 definiert ist, und R₁ folgendes repräsentiert:
(i) ein Wasserstoffatom,
(ii) ein C₁-C₄₀ Alkylradikal, linear oder verzweigt, gesättigt oder ungesättigt, das möglicherweise durch wenigstens ein Hydroxid oder ein Radikal 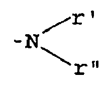 substituiert ist,
und/oder möglicherweise durch wenigstens ein Heteroatom unterbrochen ist, das unter N, O oder Si ausgewählt ist, wobei r' und r", ob identisch oder verschieden, ein Wasserstoffatom oder eine C₁-C₆ Alkylradikal sind,
(iii) 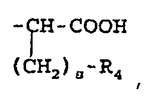 mit s 0 bis 4 ist,
(iv) 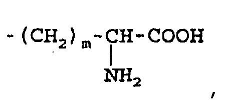 mit m 3 bis 5 ist,
wobei R₄ ein Wasserstoffatom, NH₂, OH, SH, -CHOHCH₃, - CONH₂, 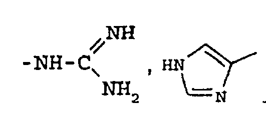 -C₆H₅ oder C₈H₅pOH bedeuten,
wobei wenigstens einer der Precursorstoffe in einem kosmetisch verträglichen Milieu vorhanden ist;
Frottieren der Fasern für ca. 2 bis 30 Minuten; und möglicherweise Spülen derselben.

21. Verfahren zur Behandlung von Keratinfasern nach Anspruch 20, **dadurch gekennzeichnet, dass** die Keratinfasern nach dem Schritt des Spülens einer Schamponierung unterzogen werden.

22. Zweiteilige Packung zur Durchführung des Verfahrens nach den Ansprüchen 20 und 21, **dadurch gekennzeichnet, dass** der erste Teil wenigstens ein nach Anspruch 20 definiertes N-Carboxyanhydrid der Formel (II) in festem Aggregatzustand oder in einem kosmetisch verträglichen Trägerstoff verdünnt enthält, und dass der zweite Teil wenigstens eine nucleophile Verbindung der nach Anspruch 20 definierten Formel (III) in festem Aggregatzustand oder in einem kosmetisch verträglichen Trägerstoff verdünnt enthält.

23. Zweiteilige Packung nach Anspruch 22, **dadurch gekennzeichnet, dass** der kosmetisch verträgliche Trägerstoff des N-Carboxyanhydrids der Formel (II) ein organisches Lösungsmittel, Wasser oder ein Gemisch aus diesen ist.

24. Zweiteilige Packung nach Anspruch 22, **dadurch gekennzeichnet, dass** der kosmetisch verträgliche Trägerstoff der nucleophilen Verbindung der Formel (III) Wasser ist.

25. Zweiteilige Packung nach einem der Ansprüche 22 bis 24, **dadurch gekennzeichnet, dass** wenigstens eine der zwei Teilen wenigstens einen herkömmlichen kosmetischen Bestandteil enthält, der nach Anspruch 19 definiert ist.

26. Verwendung wenigstens eines Polyaminosäurederivats der nach Anspruch 1 definierten allgemeinen Formel (I) in einer haarkosmetische Zusammensetzung, um den Halt und das Volumen des Haars zu verbessern.
